# EUROPÄISCHE PATENTSCHRIFT

(11) **EP 0 537 184 B1**
(45) Veröffentlichungstag und Bekanntmachung des Hinweises auf die Patenterteilung: **14.12.1994**
(21) Anmeldenummer: 91910551.0
(22) Anmeldetag: 08.06.1991
(51) Int. Cl.: C07C 69/34, C08K 5/11

(54) **MISCHESTER UND SEINE VERWENDUNG ALS GLEITMITTEL IN KUNSTSTOFF-FORMMASSEN**
MIXED ESTER AND ITS USE AS A PARTING AGENT IN PLASTIC MOULDING MATERIALS
ESTER MELANGE ET SON UTILISATION COMME AGENT DE DEMOULAGE DE MATIERES PLASTIQUES MOULABLES

(30) Priorität: 27.06.1990 DE 4020483
(43) Veröffentlichungstag der Anmeldung: 21.04.1993
(73) Patentinhaber: HOECHST AKTIENGESELLSCHAFT, 65926 Frankfurt am Main (DE)
(72) Erfinder: HEINRICHS, Franz-Leo, D-8901 Gablingen (DE); HOHNER, Gerd, D-8906 Gersthofen (DE); PIESOLD, Jan-Peter, D-8900 Augsburg (DE)
(86) Internationale Anmeldenummer: EP9101078
(87) Internationale Veröffentlichungsnummer: WO9200269

(56) Entgegenhaltungen:
- BE-A- 0 412 399
- DE-A- 1 907 768
- US-A- 2 025 612
- DATABASE WPIL, Derwent Publications,, Ltd., London, GB, Abs 91-161153; JP-A-3097789

## Beschreibung

Die Erfindung bezieht sich auf einen Mischester, der als Gleitmittel für die thermoplastische Verarbeitung von Kunststoffen, insbesondere auf der Basis von Vinylpolymeren, dient und überwiegend aus einem oligomeren komplexen Ester besteht.

Thermoplastische Kunststoffe werden in der Regel oberhalb ihres Schmelzpunktes verarbeitet. Die Art der Verarbeitung richtet sich hierbei nach den Eigenschaften des verwendeten Kunststoffs sowie nach der Form des angestrebten Endprodukts. Ganz überwiegend angewendete Verarbeitungsverfahren sind das Spritzgießen, bei welchem die Schmelze in eine Form eingespritzt und der geformte Artikel nach dem Abkühlen unter Schmelzpunkt entnommen wird; das Kalandrieren, bei welchem Folien oder Platten zwischen den Walzenpaaren eines Kalanders geformt werden, oder die verschiedenen Extrusionsverfahren, nach denen z.B. Schläuche, Profile, Rohre und ähnliche Artikel hergestellt werden.

Zahlreiche Kunststoffschmelzen zeigen ein großes Haftvermögen an den metallischen Teilen der Verarbeitungsmaschinen, woraus häufig Verarbeitungsprobleme entstehen können. Beim Spitzguß wird beispielsweise die Entnahme der Artikel aus der Form erschwert, wobei nicht nur die Gefahr ihrer Beschädigung besteht, sondern es auch zu Betriebsstörungen durch nicht entnehmbare Teile kommen kann. Bei der Folienherstellung führt ein Haften der Schmelze zu einer rauhen Oberfläche und zu Dickenschwankungen infolge von unkontrollierbaren Dehnungen beim Abzug von den Walzen. Um diesen Problemen zu begegnen, werden bei der Verarbeitung Gleit- und Formtrennmittel zugesetzt, die einen trennwirksamen Film zwischen der Polymerschmelze und der Verarbeitungsmaschine aufbauen.

Bekannt ist ein Gleitmittel für die formgebende Verarbeitung thermoplastischer Massen, welches Mischester mit Hydroxyl- bzw. Säurezahlen von 0 bis 6 aus a) aliphatischen, cycloaliphatischen und/oder aromatischen Dicarbonsäuren, b) aliphatischen Polyolen und c) aliphatischen Monocarbonsäuren mit 12 bis 30 C-Atomen im Molekül enthält (vgl. DE 1 907 768). Die bevorzugten Ester basieren auf Fettsäuren mit nicht mehr als 22 C-Atomen.

Für die große Gruppe der Vinylpolymeren und hierbei insbesondere für das Polyvinylchlorid werden zumindest bei hochwertigen Anwendungen zwei Gruppen von äußeren Gleitmitteln bevorzugt eingesetzt.

Es sind dies die verschiedenen halbsynthetischen Wachse auf Basis Montanwachs und die vorgenannten oligomeren Ester, die aus aliphatischen Dicarbonsäuren, Polyolen und Fettsäuren hergestellt werden.

Obwohl die beiden genannten Gleitmitteltypen zu den wirksamsten aller Gleitmittel gehören, vereinigen sie jeweils für sich allein betrachtet nur einen Teil der Anforderungen, die an ein ideales Gleitmittel zu stellen sind. Die Montansäurederivate zeigen eine gute Trennwirkung und verleihen insbesondere bei der PVC-Verarbeitung der Schmelze eine hohe Festigkeit und dem Endprodukt eine sehr gute Wärmeformbeständigkeit. Nachteilig ist jedoch ihre Trübungsneigung, vor allem in nicht schlagzäh modifiziertem PVC, wodurch ihre Einsatzmenge gelegentlich auf Kosten einer ausreichenden Trennwirkung stark limitiert wird.

Zudem führt die Verwendung herkömmlicher Montansäureester bei der Herstellung von PVC-Kalanderfolien zu hohen Drücken im Walzenspalt der Verarbeitungsmaschine und damit zu einer hohen Belastung (Spaltlast). Sowohl einfache Fettsäureester, wie z.B. Stearylstearat, als auch die oben erwähnten oligomeren Fettsäureester vermindern die Spaltlast bei der PVC-Verarbeitung. Nachteilig ist jedoch, daß sie zugleich die Schmelzfestigkeit und die Wärmeformbeständigkeit des PVC herabsetzen. In nicht schlagzäh modifiziertem PVC zeigen sowohl einfache als auch oligomere Fettsäureester darüberhinaus ein günstigeres Transparenzverhalten als Montansäurederivate.

Weil die zur Zeit verfügbaren anderen Gleit- und Trennmittel die Anforderungen jeweils nur zum Teil erfüllen, wird in der Regel mit Gleitmittelmischungen gearbeitet. Diese Vorgehensweise führt jedoch dazu, daß die Rezepte recht kompliziert werden und nachteilige Wechselwirkungen zwischen den zahlreichen Bestandteilen auftreten können. Zudem erfordern mehr Rezeptbestandteile eine größere Lagerhaltung und aufwendigere Dosier- und Mischeinrichtungen bei der Verarbeitung.

Es bestand daher ein Bedarf an Gleitmitteln, welche die positiven Eigenschaften der Montansäurederivate und der oligomeren Fettsäureester in sich vereinen und dabei nicht die beschriebenen Nachteile dieser Gleitmittel zeigen.

Es wurde nun gefunden, daß bestimmte Mischester, die aus Dicarbonsäuren, langkettigen Monocarbonsäuren und aliphatischen Polyolen entstehen, diese Anforderungen besonders gut erfüllen.

Die Erfindung betrifft somit einen Mischester einer langkettigen, aliphatischen Monocarbonsäure mit 24 bis 40 C-Atomen, einer aliphatischen, cycloaliphatischen oder aromatischen Dicarbonsäure mit 2 bis 12 C-Atomen und einem aliphatischen Polyol mit 3 bis 12 C-Atomen und 3 bis 6 OH-Gruppen, mit einer Säurezahl 8 - 30 mg KOH/g, einer Verseifungszahl 130 bis 220 mg KOH/g und einer Schmelzviskosität bei 90°C 200 bis 2000 mPa.s.

Der erfindungsgemäße Mischester entsteht durch Veresterung einer Dicarbonsäure und einer langkettigen Monocarbonsäure mit einem Polyol.

Die Dicarbonsäure hat 2 bis 12 C-Atome im Molekül und kann aliphatisch, cycloaliphatisch oder aromatisch sein. Beispiele für geeignete Dicarbonsäuren sind Oxalsäure, Malonsäure, Bernsteinsäure, Glutarsäure, Adipinsäure, Pimelinsäure, Korksäure, Azelainsäure, Sebacinsäure, Nonandicarbonsäure, Decandicarbonsäure, Maleinsäure, Fumarsäure, Citraconsäure, Mesaconsäure, Itaconsäure, Cyclopropandicarbonsäure, Cyclobutandicarbonsäure, Cyclopentandicarbonsäure, Camphersäure, Hexahydrophthalsäure, Phthalsäure, Terephthalsäure, Isophthalsäure. Die bevorzugte Dicarbonsäure ist Adipinsäure.

Die langkettige Monocarbonsäure hat 24 bis 40 C-Atome im Molekül und ist vorzugsweise technische Montansäure, wie sie bei der oxidativen Bleichung von Rohmontanwachs entsteht. Sie ist ein Gemisch von Monocarbonsäuren mit weit überwiegendem Anteil an C₂₆-C₃₄-Säuren (ca. 80 %) und einem geringen Anteil an Säuren mit weniger als 26 C-Atomen.

Das aliphatische Polyol hat 3 bis 12 C-Atome und 3 bis 6 OH-Gruppen im Molekül. Beispiele sind Glycerin, Diglycerin, Trimethylolpropan, Di-trimethylolpropan, Erythrit, Pentaerythrit, Dipentaerythrit, Xylit, Mannit, Sorbit. Bevorzugte Polyole sind Glycerin, Diglycerin, Trimethylolpropan, Di-trimethylolpropan, Pentaerythrit und Dipentaerythrit.

Die Herstellung des erfindungsgemäßen Mischesters erfolgt nach an sich bekannten Veresterungsverfahren, indem man beispielsweise eine langkettige aliphatische Monocarbonsäure mit einem zwei oder mehrwertigen Polyol so verestert, daß der erhaltene Teilester noch mehrere freie Hydroxylgruppen enthält und daß man dann diese Hydroxylgruppen mit einer entsprechenden Dicarbonsäure umsetzt. Man kann aber auch zunächst einen Teil der Hydroxylgruppen eines Polyols mit einer Dicarbonsäure verestern und danach die restlichen freien Hydroxylgruppen des erhaltenen Teilesters mit einer längerkettigen aliphatischen Monocarbonsäure umsetzen.

Die Umsetzung wird so geführt, daß der fertige Mischester eine Säurezahl von 8 bis 30, vorzugsweise 8 bis 20 mg KOH/g und eine Verseifungszahl von 130 bis 220, vorzugsweise 130 bis 200 mg KOH/g besitzt.

Im erfindungsgemäßen Mischester sind Dicarbonsäure, langkettige Monocarbonsäure und Polyol im Äquivalentverhältnis (0,4-1,0): (1,5-2,5): (2,5-3,5), vorzugsweise im Äquivalentverhältnis (0,7-0,9): (1,8-2,2): (2,8-3,2) enthalten. Besonders bevorzugt ist ein Mischester aus Adipinsäure, Montansäure und Trimethylolpropan im ungefähren Äquivalentverhältnis 0,8:2:3.

Der erfindungsgemäße Mischester ist ein fester Stoff und besitzt eine Schmelzviskosität bei 90°C von 200 bis 2000, vorzugsweise 200 bis 800 mPa.s.

Der Mischester wird als Gleitmittel in thermoplastischen Kunststoff-Formmassen eingesetzt, beispielsweise in Polyvinylchlorid, Mischpolymerisaten des Vinylchlorids, welche durch die bekannten Verfahren hergestellt werden können (z.B. Suspensions-, Masse-, Emulsionspolymerisation), Mischpolymeren des Vinylchlorids mit bis zu 30 Gew.-% an Comonomeren wie z.B. Vinylacetat, Vinylidenchlorid, Vinylether, Acrylnitril, Acrylsäureester, Maleinsäuremono-oder -diestern oder Olefinen, sowie Pfropfpolymerisaten des Polyvinylchlorids und Polyacrylnitril.

Bevorzugt sind Polyvinylchlorid, Polyvinylidenchlorid, Ethylen-Vinylacetat-Copolymere, Polyacrylnitril, Copolymere aus Vinylchlorid und Vinylacetat sowie daraus abgeleitete Pfropfpolymere oder Mischungen der vorgenannten Thermoplaste.

Die Zugabemenge beträgt 0,05 bis 5 Gew.-%, bezogen auf das Polymere. Basiert die Formmasse auf M- oder S-PVC, so beträgt die Zugabemenge vorzugsweise 0,05 bis 1 Gew.-%, basiert sie auf E-PVC, so ist die Menge vorzugsweise 1,0 bis 5, insbesondere 2 bis 4 Gew.-%, jeweils bezogen auf das Polymere.

Das Einmischen des erfindungsgemäßen Mischesters in die Polymeren geschieht auf die übliche Weise während der Herstellung der Formmassen.

Neben dem erfindungsgemäßen Mischester kann die Kunststoff-Formmasse zusätzlich Füllstoffe, Wärmestabilisatoren, Lichtschutzmittel, Antistatika, Flammschutzmittel, Verstärkungsstoffe, Pigment- und/oder Farbstoffe, Verarbeitungshilfsmittel, Gleit- und Trennmittel, Schlagzähmacher, Antioxidantien, Treibmittel oder optische Ainfheller in den üblichen Mengen enthalten.

Die erfindungsgemäßen Mischester reduziert die auftretende Spaltlast bei der Herstellung von Kalanderfolien, wobei er in seiner Wirkung die bekannten Mischester auf Fettsäurebasis übertrifft. Zugleich verhindert er die Haftung der Formmasse an den Walzen und zeigt im Vergleich zu den handelsüblichen Montanwachsen ein besseres Transparenzverhalten.

Die nachfolgenden Beispiele sollen die Erfindung erläutern.

### Beispiel 1

### Montansäurekomplexester 1

402,7 g Montansäure (SZ = 139,2 mg KOH/g) wurden in einem Wittschen Topf aufgeschmolzen. Unter Rühren wurden zunächst 67,1 g Trimethylolpropan und danach 1,32 cm³ Bintylzinntris-2-ethylhexanoat als Katalysator zugegeben. Unter Stickstoffatmosphäre wurde die Mischung unter fortgesetztem Rühren auf 125°C erhitzt, wobei das bei der Veresterungsreaktion gebildete Wasser fortwährend abdestilliert wurde. Als die SZ der Mischung unter 10 mg KOH/g gesunken war, wurden 29,2 g Adipinsäure zugesetzt. Unter den gleichen Bedingungen wurde die Reaktion fortgesetzt, bei die SZ wiederum unter 10 mg KOH/g gefallen war. Danach wurden zur Farbverbesserung 2,5 cm³ 35%iges Wasserstoffperoxid zingesetzt, der Ansatz wurde weitere 15 Minuten gerührt und dann getrocknet und filtriert.

Man erhielt ein gelbliches, hartes Wachs mit einer Säurezahl von 8,3 mg KOH/g, einer Verseifungszahl von 180 mg KOH/g und einer Schmelzviskosität von 573 mPa.s (90°C).

### Beispiel 2

### Montansäurekomplexester 2

402,7 g Montansäure (SZ = 139,2 mg KOH/g) wurden wie im Beispiel 1 aufgeschmolzen und bei 80°C mit 67,1 g Trimethylolpropan, 29,2 g Adipinsäure, 2,1 cm³ hypophosphoriger Säure und 0,37 cm³ Methansulfonsäure versetzt. Unter Rühren wurde die Mischung auf 120°C erhitzt, wobei Reaktionswasser abdestillierte. Nachdem die Säurezahl unter 10 mg KOH/g abgefallen war, wurden 7,7 cm³ 10%ige NaOH zugesetzt, um die als Katalysatoren zugesetzten Säuren zu neutralisieren. Danach wurde das Produkt mit 2,5 cm³ Wasserstoffperoxid gebleicht. Nach dem Trocknen und Filtrieren erhielt man ein hellgelbes Wachs mit einer Säurezahl von 10,5 mg KOH/g, einer Verseifungszahl von 172 mg KOH/g und einer Schmelzviskosität von 459 mPa.s (90°C).

### Beispiel 3

### Montansäurekomplexester 3

125,0 kg Montansäure (SZ = 137 mg KOH/g) wurden aufgeschmolzen und auf 80°C erwärmt. Unter Rühren wurden 20,6 kg Trimethylolpropan und 400 g p-Toluolsulfonsäure zugesetzt. Die Temperatur wurde auf 130°C erhöht, wobei das Reaktionswasser abdestillierte. Nach etwa 100 Minuten war die Säurezahl des Ansatzes unter 10 mg KOH/g gefallen. Nach dem Zusatz von 8,94 kg Adipinsäure wurde die Mischung weitere 2 Stunden unter Rühren und Abdestillieren von Reaktionswasser erhitzt.

Zur Neutralisation der Katalysatorsäure wurden 600 cm³ 10%ige NaOH zugesetzt, anschließend wurde das Produkt durch Zugabe von 700 cm³ 35%igem Wasserstoffperoxid gebleicht. Nach der Filtration erhielt man ein gelbes Wachs mit einer Säurezahl von 19,0 mg KOH/g, einer Verseifungszahl von 187 mg KOH/g und einer Schmelzviskosität von 202 mPas (90°C).

In den folgenden Beispielen wurden folgende Produkte als Vergleich zum Stand der Technik verwendet:
A Oligomerer Fettsäureester: Mischester aus Stearinsäuren, Pentaerythrit und Adipinsäure
B Glycerinmontanat
C Ethylenglykolmontanat

### Beispiel 4

Der Montansäurekomplexeser 3 wurde in folgendem Rezept auf einem Meßwalzwerk mit einem Walzendurchmesser von 150 mm und einer Ballenbreite von 350 mm (Hersteller Fa. Collins) geprüft. Die Auftragsmenge betrug 200 g. Die elektrisch beheizten Walzen waren auf eine Temperatur von 195°C eingestellt. Ihre Drehzahl betrug 15/20 Upm. Hierbei wurden die auftretenden Lagerkräfte (Spaltlast) P_{K} (in kN) gemessen. Das Meßwalzwerk verfügte zudem über eine automatische Wendevorrichtung für das Walzfell. Die Kraft, welche für diesen Wendevorgang benötigt wird, ist ein Maß für die Haftkraft des Walzfells auf den Chromwalzen (Haftkraft F_{K} : in N).

| | |
|---|---|
| S-PVC (K-Wert ca. 60) | 100 Gew.-Teile |
| Thiozinnstabilisator | 1,5 Gew.-Teile |
| MBS-Schlagzähmacher | 4,0 Gew.-Teile |
| acrylathaltiges Verarbeitungshilfsmittel | 1,0 Gew.-Teile |
| epox. Sojabohnenöl | 0,5 Gew.-Teile |
| Glycerinmonostearat | 0,8 Gew.-Teile |
| Prüfprodukt | 0,3 Gew.-Teile |

| Versuch | Prüfprodukt |
|---|---|
| I | oligomerer Montansäureester 3 |
| II | oligomerer Fettsäureester A |
| III | Glycerinmontanat B |
| IV | Ethylenglykolmontanat C |

Kraftangabe in F_{K} in N, P_{K} in kN

| Versuch | I | | II | | III | | IV | |
|---|---|---|---|---|---|---|---|---|
| Zeit (sec) | F_{K} | P_{K} | F_{K} | P_{K} | F_{K} | P_{K} | F_{K} | P_{K} |
| 120 | 174 | 7,5 | 170 | 7,9 | 176 | 7,7 | 178 | 8,0 |
| 240 | 175 | 7,7 | 173 | 7,9 | 174 | 8,1 | 177 | 8,1 |
| 480 | 172 | 7,7 | 174 | 8,0 | 176 | 7,9 | 181 | 8,1 |
| 960 | 176 | 7,9 | 179 | 7,9 | 178 | 8,1 | 318 | 8,1 |

Auf einem Walzwerk bei 190°C und 15/20 Upm wurden die klebfreie Zeit sowie die Endstabilität dieser Mischungen geprüft.

| Prüfprodukt | klebfreie Zeit (min) | Endstabilität (min) |
|---|---|---|
| Montansäurekomplexester 3 | 42 | 48 |
| Fettsäurekomplexester A | 33 | 47 |
| Glycerinmontanat B | 37 | 46 |
| Ethylenglykolmontanat C | 22 | 47 |

### Beispiel 5

| Rezept | |
|---|---|
| S-PVC (K-Wert ca. 60) | 100 Gew.-Teile |
| Thiozinnstabilisator | 1,4 Gew.-Teile |
| MBS-Schlagzähmacher | 5,0 Gew.-Teile |
| acrylathaltiges Verarbeitungshilfsmittel | 1,0 Gew.-Teile |
| Glycerinmonooleat | 0,9 Gew.-Teile |
| Prüfprodukt | 0,3 Gew.-Teile |

Wie im Beispiel 4 beschrieben wurden auf einem Meßwalzwerk die Haftkraft F_{K} und die Spaltlast P_{K} gemessen.

| Versuch | Prüfprodukt |
|---|---|
| I | oligomerer Montansäureester 3 |
| II | oligomerer Fettsäureester A |
| III | Glycerinmontanat B |

| Versuch | I | | II | | III | |
|---|---|---|---|---|---|---|
| Zeit (sec) | P_{K} | F_{K} | P_{K} | F_{K} | P_{K} | F_{K} |
| 60 | 7,7 | 171 | 8,1 | 179 | 8,1 | 179 |
| 120 | 7,9 | 175 | 8,3 | 174 | 8,1 | 176 |
| 180 | 7,9 | 175 | 8,1 | 174 | 8,1 | 171 |
| 240 | 7,9 | 174 | 8,2 | 177 | 8,2 | 173 |

Auf dem Walzwerk wurden folgende Werte bestimmt (190°C; 15/20 Upm)

| Versuch | klebfreie Zeit (min) | Endstabilität (min) |
|---|---|---|
| I | 26 | 35 |
| II | 22 | 40 |
| III | 20 | 30 |

### Beispiel 6

Die Montansäurekomplexester 1 und 2 wurden in folgendem Rezept auf ihr Transparenzverhalten geprüft.

| | |
|---|---|
| S-PVC (K-Wert ca. 60) | 100 Gew.-Teile |
| acrylathaltiges Verarbeitungshilfsmittel | 1,0 Gew.-Teile |
| Octylzinnstabilisator | 1,5 Gew.-Teile |
| Glycerinmonooleat | 0,3 Gew.-Teile |
| Prüfprodukt | 0,6 Gew.-Teile |

Diese Mischungen wurden auf einem Walzwerk bei 190°C plastiziert, danach wurden 0,5 und 2,0 mm Plättchen gepresst. Diese wurden mit einem Transparenzmeßgerät im neutralgrauen Licht geprüft.

| Prüfprodukt | Transparenz in % | |
|---|---|---|
| | 0,5 mm | 2,0 mm |
| Montansäurekomplexester 1 | 85,0 | 70,2 |
| Montansäurekomplexester 2 | 86,4 | 75,7 |
| Fettsäurekomplexester A | 84,4 | 74,4 |
| Glycerinmontanat B | 75,9 | 49,4 |
| Ethylenglykolmontanat C | 73,9 | 48,5 |

### Beispiel 7

In dem folgenden Rezept wurden die Montansäurekomplexester 1 und 3 auf ihre Trennwirkung geprüft:

| | |
|---|---|
| M-PVC (K-Wert ca. 57) | 100 Gew.-Teile |
| MBS-Schlagzähmacher | 8 Gew.-Teile |
| acrylathaltiges Verarbeitungshilfsmittel | 1,2 Gew.-Teile |
| Thiozinnstabilisator | 1,6 Gew.-Teile |
| epox. Sojabohnenöl | 1,0 Gew.-Teile |
| Glycerinmonooleat | 0,3 Gew.-Teile |
| Prüfprodukt | 0,6 Gew.-Teile |

Die Prüfung erfolgte auf einem Walzwerk bei 190°C und 15/20 Upm. Die Prüfung wurde abgebrochen, wenn sich das Walzfell braun verfärbte.

| Prüfprodukt | klebfreie Zeit | Endstabilität |
|---|---|---|
| Montansäurekomplexester 1 | 37 | 39 |
| Montansäurekomplexeter 3 | 34 | 36 |
| Fettsäurekomplexester A | 28 | 33 |
| Glycerinmontanat B | 30 | 33 |
| Ethylenglykolmontanat C | 14 | 35 |

Die Beispiele 4 und 5 zeigen, daß durch den oligomeren Montansäureester 3 eine deutliche Reduzierung der Spaltlast erfolgt. Die Beispiele 4 bis 7 zeigen außerdem, daß die oligomeren Montansäureester 1, 2 und 3 darüber hinaus im Hinblick auf die Trennwirkung und die Transparenz Vorteile aufweisen.

## Patentansprüche

1. Mischester einer langkettigen, aliphatischen Monocarbonsäure mit 24 bis 40 C-Atomen, einer aliphatischen, cycloaliphatischen oder aromatischen Dicarbonsäure mit 2 bis 12 C-Atomen und einem aliphatischen Polyol mit 3 bis 12 C-Atomen und 3 bis 6 OH-Gruppen, mit einer Säurezahl von 8 bis 30 mg KOH/g, einer Verseifungszahl 130 bis 220 mg KOH/g und einer Schmelzviskosität bei 90°C von 200 bis 2000 mPa.s.

2. Mischester nach Anspruch 1, dadurch gekennzeichnet, daß die langkettige aliphatische Monocarbonsäure technische Montansäure ist.

3. Mischester nach Anspruch 1, dadurch gekennzeichnet, daß das Polyol Glycerin, Diglycerin, Trimethylolpropan, Di-trimethylolpropan, Pentaerythrit oder Dipentaerythrit ist.

4. Mischester nach Anspruch 1, dadurch gekennzeichnet, daß er aus Montansäure, Adipinsäure und Trimethylolpropan entstanden ist.

5. Verwendung des Mischesters nach Anspruch 1 als Gleitmittel für die Verarbeitung thermoplastischer Kunststoff-Formmassen.

6. Thermoplastische Kunnststoff-Formmasse, dadurch gekennzeichnet, daß sie 0,05 bis 5.% eines Mischesters nach Anspruch 1 enthält.

7. Thermoplastische Kunststoff-Formmasse nach Anspruch 6, dadurch gekennzeichnet, daß sie auf Polyvinylchlorid, Polyvinylidenchlorid, Ethylen-Vinylacetat-Copolymeren, Polyacrylnitril, Pfropf- oder Copolymeren aus Vinylchlorid und Vinylacetat oder Mischungen der angegebenen Thermoplasten basiert.

8. Thermoplastische Kunststoff-Formmasse nach Anspruch 6, dadurch gekennzeichnet, daß sie zusätzlich Füllstoffe, Wärmestabilisatoren, Lichtschutzmittel, Antistatika, Flammschutzmittel, Verstärkungsstoffe, Pigment- und/oder Farbstoffe, Verarbeitungshilfsmittel, Gleit- und Trennmittel, Schlagzähmacher, Antioxidantien, Treibmittel oder optische Aufheller enthält.

## Claims

1. A mixed ester of a long-chain, aliphatic monocarboxylic acid having 24 to 40 carbon atoms, an aliphatic, cycloaliphatic or aromatic dicarboxylic acid having 2 to 12 carbon atoms and an aliphatic polyol having 3 to 12 carbon atoms and 3 to 6 OH groups, which has an acid number of from 8 to 30 mg of KOH/g, a hydrolysis number of from 130 to 220 mg of KOH/g and a melt viscosity at 90°C of from 200 to 2000 mPa.s.

2. A mixed ester as claimed in claim 1, wherein the long-chain, aliphatic monocarboxylic acid is technical-grade montanic acid.

3. A mixed ester as claimed in claim 1, wherein the polyol is glycerol, diglycerol, trimethylolpropane, ditrimethylolpropane, pentaerythritol or dipentaerythritol.

4. A mixed ester as claimed in claim 1, produced from montanic acid, adipic acid and trimethylolpropane.

5. The use of a mixed ester as claimed in claim 1 as a lubricant for the processing of thermoplastic molding compositions.

6. A thermoplastic molding composition which contains from 0.05 to 5% of a mixed ester as claimed in claim 1.

7. A thermoplastic molding composition as claimed in claim 6 which is based on polyvinyl chloride, polyvinylidene chloride, ethylene-vinyl acetate copolymers, polyacrylonitrile, graft polymers or copolymers of vinyl chloride and vinyl acetate, or mixtures of said thermoplastics.

8. A thermoplastic molding composition as claimed in claim 6, which additionally contains fillers, heat stabilizers, light screens, antistatics, flameproofing agents, reinforcing agents, pigments, dyes, processing assistants, lubricants, mold-release agents, impact modifiers, antioxidants, blowing agents or optical brighteners.

## Revendications

1. Ester mixte d'un acide monocarboxylique aliphatique à longue chaîne comportant 24 à 40 atomes de carbone, d'un acide dicarboxylique, aliphatique, cycloaliphatique ou aromatique comportant 2 à 12 atomes de carbone et d'un polyol aliphatique comportant 3 à 12 atomes de carbone et 3 à 6 groupes OH, ayant un indice d'acide de 8 à 30 mg de KOH/g, un indice de saponification de 130 à 220 mg/KOH et une viscosité, à l'état fondu à 90°C, de 200 à 2000 m Pa.s.

2. Ester mixte selon la revendication l, caractérisé en ce que l'acide monocarboxylique aliphatique à longue chaîne est de l'acide montanique, de qualité technique.

3. Ester mixte selon la revendication 1, caractérisé en ce que le polyol est le glycérol, le diglycérol, le triméthylol propane, le di-triméthylol propane, le pentaérythritol ou le dipentaérythritol.

4. Ester mixte selon la revendication 1, caractérisé en ce qu'il est obtenu à partir d'acide montanique, d'acide adipique et de triméthylol propane.

5. Utilisation de l'ester mixte selon la revendication 1 comme lubrifiant pour le traitement de transformation de compositions de matière thermoplastique à mouler.

6. Composition de matière thermoplastique à mouler, caractrisée en ce qu'elle contient 0,05 à % 5 % d'un ester mixte selon la revendication 1.

7. Composition de matière thermoplastique à mouler selon la revendication 6, caractérisée en ce qu'elle est à base de poly(chlorure de vinyle), de poly(chlorure de vinylidène), de copolymères d'éthylène/acétate de vinyle, de polyacrylonitrile, de polymère(s) greffé(s) ou de copolymères du chlorure de vinyle et de l'acétate de vinyle ou de mélanges des matières thermoplastiques indiquées.

8. Composition de matière thermoplastique à mouler selon la revendication 6,caractérisée en ce qu'elle contient en outre des charges, des agents de stabilisation à l'égard de la chaleur, des agents de protection à l'égard des effets de la lumière, des anti(électricté)statique, des agents de protection contre la flamme, des matières d'armatures de renforcement, du pigment et/ou des colorants, des adjuvants facilitant la mise en oeuvre, des lubrifiants et des agents de séparation ou de démoulage, des agents conférant de la résilience ou de la résistance aux chocs, des anti-oxydants, des porogènes ou des agents d'avivage optique.
